# EUROPEAN PATENT APPLICATION

(11) **EP 4 338 714 A1**
(43) Date of publication of application: **20.03.2024**
(21) Application number: 22827386.8
(22) Date of filing: 08.06.2022
(51) Int. Cl.: A61F 2/90

(54) **STENT AND DRUG-LOADED STENT**

(30) Priority: 22.06.2021 CN 202110693975
(71) Applicant: MicroPort NeuroTech (Shanghai) Co., Ltd., Shanghai 201318 (CN)
(72) Inventor: HOU, Juan, Shanghai 201318 (CN); SONG, Zhihao, Shanghai 201318 (CN); TIAN, Hao, Shanghai 201318 (CN); LEI, Wenbin, Shanghai 201318 (CN)
(74) Representative: Marks & Clerk LLP
(86) International application number: PCT/CN2022/097719
(87) International publication number: WO 2022/267890

(57) **Abstract**

The present invention provides a stent and a drug-loaded stent. The stent includes at least one stent mesh, wherein each stent mesh comprises a plurality of stent struts sequentially connected circumferentially around the stent mesh, wherein the plurality of the stent struts are sequentially connected end to end, and a joint is formed at the connected ends of adjacent stent struts. The stent mesh is configured to expand or collapse as a result of widening or narrowing of angles at the joints. Each stent strut includes at least one main section and at least one broadened section. The main section has a width smaller than that of the broadened section. In each stent mesh, the broadened sections of adjacent stent struts are staggered along an axis of the stent mesh. The drug-loaded stent is of a similar structure. The different widths of the broadened sections and the main sections, as well as the staggered arrangement of the broadened sections, enable reasonable space utilization and achieve a good tradeoff between metal coverage and flexibility of the stent.

## Description

### TECHNICAL FIELD

The present invention pertains to the field of medical devices. In particular, it relates to a stent and a drug-loaded stent

### BACKGROUND

Intracranial atherosclerotic stenosis (ICAS) is a significant cause of the occurrence, development and recurrence of ischemic stroke. Clinical intervention focuses on ICAS cases with symptoms, and principal therapeutic measures include antiplatelet medication, angioplasty and traditional surgery. Although antiplatelet medication is a classic therapy for symptomatic ICAS, it is associated with a high incidence of stroke. Traditional surgery is highly risky and therefore seldom employed.

As a result of developments in intracranial intervention, at present, angioplasty has become an important method for symptomatic ICAS treatment. Angioplasty procedures include dilation with a bare metal stent, a simple balloon, a coronary drug-coated stent or the like. However, bare metal stent or simple balloon based dilation is associated with a risk of restenosis, and coronary drug-coated stents are substantially balloon-expanded drug-coated stents. Such stents have a great outer diameter dimension when loaded in a delivery system, which makes delivery to a target site difficult, in particular through a tortuous vascular site or complex lesion site. In contrast, self-expanding stents designed for delivery by a microcatheter can be more easily delivered to a target site because of a reduced outer diameter dimension when loaded in a delivery system.

Differing from extracranial arteries, the physiological structure of an intracranial artery is characterized by: 1) great physiological curvature, as well as significant physiological tortuosity, in particular when it is arteriosclerotic usually as a consequence of stenosis; and 2) a thin and inelastic outer layer, which is located in the subarachnoid space without support from tissue and therefore exhibits poor resistance to mechanical damage. Once a drug escapes from an intracranial artery through a rupture in its wall, or permeates therethrough, it tends to spread through the cerebrospinal fluid across the entire brain and even reach the spinal cord. Accordingly, stents for use in intracranial arteries are desired to have suitable radial strength. A stent with excessive radial strength may be difficult to deliver to a target site, or may cause damage to a blood vessel during use. On the other hand, a stent with insufficient radial strength may be not able to adequately support a stenotic plaque in a blood vessel. Due to great physiological curvature of an intracranial artery, it is usually difficult for a stent to be delivered to a lesion side therein. Moreover, even when the stent manages to reach the site, if it cannot well comply with the geometry of the blood vessel there during expansion, the blood vessel tends to be stretched, or damage may be caused thereto. Therefore, a stent for use in an intracranial artery is desired to have good flexibility and compliance, which allow the stent to be easily delivered to a target site in the artery and adapt itself to the geometry of the blood vessel there. Further, in order to be able to cover a plaque in a desired manner, a stent is required to have sufficient metal coverage. However, for an intracranial blood vessel, which is vulnerable and lacks elasticity, high metal coverage tends to cause excessive radial strength and poor flexibility. High radial strength may lead to damage to the intracranial blood vessel and hence possible in-stent restenosis.

Thus, conventional stents fail to provide both sufficient metal coverage and desirable flexibility.

### SUMMARY OF THE INVENTION

It is an object of the present invention to provide a stent, which can overcome the problem of failing to provide both good metal coverage and desirable flexibility associated with conventional stents.

To this end, a stent is provided herein, wherein the strut comprises at least one stent mesh each comprising a plurality of stent struts sequentially connected circumferentially around the stent mesh, the plurality of the stent struts sequentially connected end to end, and a joint formed at the connected ends of adjacent stent struts, each stent mesh configured to expand or collapse as a result of widening or narrowing of the angle at the joint,
wherein each stent strut comprises at least one main section and at least one broadened section that are alternately arranged with the at least one main section, the main section having a width smaller than that of the broadened section; in each stent mesh, the broadened sections of adjacent stent struts are staggered along an axis of the stent mesh; and when the angle at each joint is minimized, at least one gap is provided between the broadened sections of the adjacent stent struts along the axis.

Optionally, when the angle at the joint is minimized, a length of the gap may account for 0 to 90% of a length of the stent strut.

Optionally, when the angle at the joint is minimized, the length of the gap may account for 10% to 75% of the length of the stent strut.

Optionally, when the angle at the joint is minimized, the length of the gap may account for 20% to 50% of the length of the stent strut.

Optionally, when the angle at the joint is minimized, the gap may have a length of 0.1 mm to 0.3 mm.

Optionally, the angles at each joint may range from 0° to 140°.

Optionally, when the angle at each joint is in the range of 0° to 5°, a metal coverage of the stent mesh may range from 30% to 99%.

Optionally, when the angle at each joint is in the range of 5° to 30°, a metal coverage of the stent mesh may range from 5% to 90%.

Optionally, when the angle at each joint is in the range of 30° to 90°, metal coverage of the stent mesh may range from 4% to 15%.

Optionally, when the angle at each joint is in the range of 30° to 90°, the metal coverage of the stent mesh may range from 8% to 15%.

Optionally, when the angle at each joint is in the range of 90° to 140°, a metal coverage of the stent mesh may range from 3% to 12%.

Optionally, when the angle at each joint is in the range of 0° to 5°, a metal coverage of the stent may range from 20% to 60%.

Optionally, when the angle at each joint is in the range of 5° to 30°, a metal coverage of the stent may range from 5% to 45%.

Optionally, when the angle at each joint is in the range of 30° to 90°, a metal coverage of the stent may range from 3% to 15%.

Optionally, when the angle at each joint is in the range of 90° to 140°, a metal coverage of the stent may range from 2% to 15%.

Optionally, the stent may comprise a radial strength of 1 kPa to 300 kPa.

Optionally, at least one of the stent strut may comprise two main sections and one broadened section located between the two main sections.

Optionally, at least one of the stent mesh may comprise 8 to 24 stent struts.

Optionally, at least one broadened section may comprise a cavity.

Optionally, the broadened section may comprise 1 to 10 cavities.

Optionally, a longitudinal cross-sectional shape of the cavity may comprise at least one of an arcuate shape, a quadrilateral shape and a triangular shape.

Optionally, a transverse cross-sectional shape of the cavity may comprise at least one of a circular shape, an elongate shape, a polygonal shape, a corrugated shape, an annular shape and an irregular shape.

Optionally, the cavity may be configured for a drug or radiopaque agent to be filled therein.

Optionally, when the angle at the joint is minimized, in adjacent stent struts connected to the same joint, the broadened section of one of the stent struts may not overlap with the main section of the other one of the stent struts.

Optionally, in at least one of stent strut, the broadened section may have margins of the same or different widths beyond the main section at opposite sides of the stent strut along a lengthwise direction thereof.

Optionally, in at least one of stent strut, the broadened section may be flush with the main section at one side of the stent strut along a lengthwise direction thereof.

Optionally, in an expanded configuration of the stent, adjacent stent struts connected to the same joint may form a V-shaped structure, wherein the sides of the adjacent stent struts, at which the main sections are flush with the broadened sections, are both located at inner or outer side of the V-shaped structure.

Optionally, the stent may comprise at least two stent meshes that are axially connected.

Optionally, the stent may comprise at least one linking strut, wherein the joints in adjacent stent meshes are connected through the at least one linking strut.

Optionally, a shape of the linking struts may comprise at least one of a linear shape, a corrugated shape, a serrated shape, a circular shape, an annular shape, a "Ω"-like shape and an "S"-like shape.

A drug-loaded stent is provided herein, wherein the drug-loaded stent comprises at least one stent mesh each comprising a plurality of stent struts sequentially connected circumferentially around the stent mesh, the plurality of the stent struts sequentially connected end to end, and a joint is formed at the connected ends of adjacent stent struts, each stent mesh configured to expand or collapse as a result of widening or narrowing of the angle at the joint,
wherein each stent strut comprises at least one main section and at least one broadened section that are alternately arranged with the at least one main section, the main section having a width smaller than that of the broadened section; in each stent mesh, the broadened sections of adjacent stent struts are staggered along an axis of the stent mesh; the at least one broadened section comprises a cavity configured for a drug to be filled therein; and when the angles at the joints are minimized, at least one gap is provided between the broadened sections of adjacent stent struts along the axis.

The stent provided herein includes at least one stent mesh each including a plurality of stent struts, which are connected end to end circumferentially around the stent mesh and a joint formed at the connected ends of adjacent stent struts. The stent mesh is configured to expand or collapse as a result of widening or narrowing of angles at the joints. Each stent strut includes main section(s) and broadened section(s). The main sections have a width smaller than that of the broadened sections. In each stent mesh, the broadened sections of adjacent stent struts are staggered along an axis of the stent mesh. The different widths of the broadened sections and the main sections, the staggered arrangement of the broadened sections and the gaps between the broadened sections of adjacent stent struts impart to the present invention at least one of the following advantages over the prior art:
1. The axially spaced gaps between the broadened sections of adjacent stent struts can prevent interference between the broadened sections when the stent is being flexed, resulting in improved flexibility of the stent. In this way, the stent achieves a good tradeoff between metal coverage and flexibility, solving the problem of failing to provide both good metal coverage and desirable flexibility associated with conventional stents.
2. The axially spaced gaps between the broadened sections of adjacent stent struts impart increased compliance to the stent, which allows the stent to better comply with the geometry of a site in a blood vessel, without stretching the blood vessel or causing damage thereto.
3. Reasonable space utilization and a good tradeoff between metal coverage and radial strength are achieved.
4. The staggered arrangement of the broadened sections allows the stent to have a compact size when in a collapsed configuration, which facilitates passage of the stent through a lesion site.
5. Since the broadened sections have a relatively large surface area, cavities can be formed therein, in which a drug or radiopaque agent can be filled to increase the stent's drug loading capacity or radiopacity.

### BRIEF DESCRIPTION OF THE DRAWINGS

Those of ordinary skill in the art would appreciate that the accompanying drawings are provided to facilitate a better understanding of the present invention and do not limit the scope thereof, in which:
Fig. 1a is a schematic partial view of a stent mesh according to an embodiment of the present invention;
Fig. 1b is a schematic diagram showing the stent mesh of Fig. 1a in a collapsed configuration;
Fig. 2 is a schematic partial view of a stent mesh according to another embodiment of the present invention;
Fig. 3a is a schematic partial view of a stent mesh according to yet another embodiment of the present invention;
Fig. 3b is a schematic diagram showing the stent mesh of Fig. 3a in a collapsed configuration;
Fig. 4a is a schematic diagram showing a broadened section according to an embodiment of the present invention;
Fig. 4b is a schematic diagram showing a broadened section according to another embodiment of the present invention;
Fig. 4c is a schematic diagram showing a broadened section according to yet another embodiment of the present invention;
Fig. 5a schematically illustrates a first optional implementation of a cavity pattern according to the present invention;
Fig. 5b schematically illustrates a second optional implementation of a cavity pattern according to the present invention;
Fig. 5c schematically illustrates a third optional implementation of a cavity pattern according to the present invention;
Fig. 5d schematically illustrates a fourth optional implementation of a cavity pattern according to the present invention;
Fig. 5e schematically illustrates a fifth optional implementation of a cavity pattern according to the present invention;
Fig. 6 is a schematic partial view of a stent according to an embodiment of the present invention;
Fig. 7a is a schematic partial view of a stent mesh according to the present invention, particularly showing an implementation of joints therein;
Fig. 7b is a schematic diagram showing the stent mesh of Fig. 7a in a collapsed configuration;
Fig. 8a is a schematic partial view of a stent mesh according to the present invention, particularly showing another implementation of joints therein;
Fig. 8b is a schematic diagram showing the stent mesh of Fig. 8a in a collapsed configuration;
Fig. 9a is a schematic partial view of a stent mesh according to the present invention, particularly showing yet another implementation of joints therein; and
Fig. 9b is a schematic diagram showing the stent mesh of Fig. 9a in a collapsed configuration.

In figures,
100, a stent mesh; 110, a stent strut; 120, a joint; 130, a linking strut; 101, a main section; 102, a broadened section; 103, a main segment; 104, a transition segment; 105, a cavity; 121, a bend; 140, a gap; 200, a widthwise direction; and 300, a lengthwise direction.

### DETAILED DESCRIPTION

Objects, advantages and features of the present invention will become more apparent upon reading the following more detailed description of the present invention with reference to the accompanying drawings. Note that the figures are provided in a very simplified form not necessarily drawn to exact scale and for the only purpose of facilitating easy and clear description of the embodiments. In addition, the structures shown in the figures are usually a part of actual structures. In particular, as the figures tend to have distinct emphases, they are often drawn to different scales.

As used herein, the singular forms "a", "an" and "the" include plural referents. Herein, the term "or" is generally employed in the sense of "and/or", "several" is generally employed in the sense of "at least one", and "at least two" is generally employed in the sense of "two or more". Additionally, the use of the terms "first", "second" and "third" herein is intended for illustration only and is not to be construed as denoting or implying relative importance or as implicitly indicating the numerical number of the referenced item. Accordingly, defining an item with "first", "second" or "third" is an explicit or implicit indication of the presence of one or at least two of the items. As used herein, the term "proximal end" generally refers to an end closer to an operator, and the term "distal end" generally refers to an end closer to a patient. The terms "one end" and "the other end", as well as "proximal end" and "distal end", are generally used to refer to opposite end portions including the opposite endpoints, rather than only to the endpoints. The terms "mounting", "coupling" and "connection" should be interpreted in a broad sense. For example, a connection may be a permanent, detachable or integral connection, a mechanical and electrical connection, a direct or indirect connection with one or more intervening media, or an internal communication or interaction between two elements. As used herein, when an element is referred to as being "disposed on" another element, this is generally intended to only mean that there is a connection, coupling, engagement or transmission relationship between the two elements, which may be either direct or indirect with one or more intervening elements, and should not be interpreted as indicating or implying a particular spatial position relationship between the two elements, i.e., the element may be located inside, outside, above, under, beside, or at any other location with respect to the other element, unless the context clearly dictates otherwise. Those of ordinary skill in the art can understand the specific meanings of the above-mentioned terms herein, depending on their context.

In principle, the present invention seeks to provide a stent, which can overcome the problem of failing to provide both good metal coverage and desirable flexibility associated with conventional stents.

The invention will be described below with reference to the accompanying drawings.

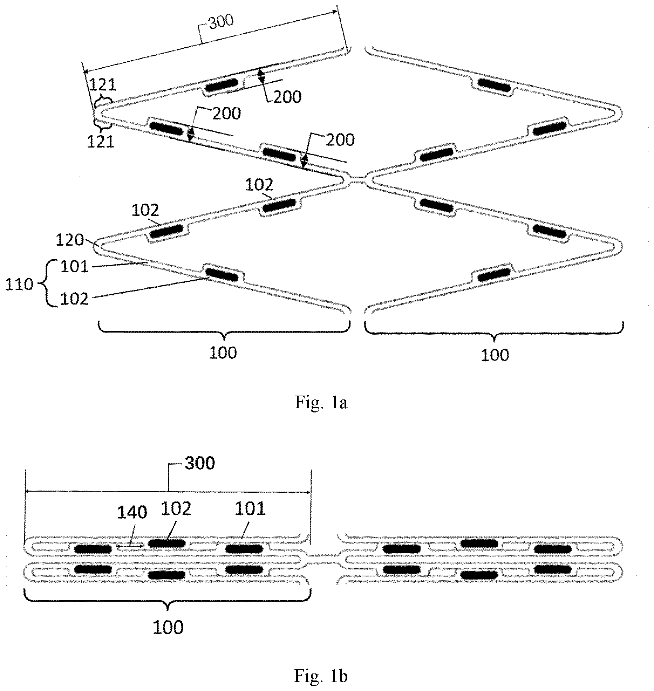

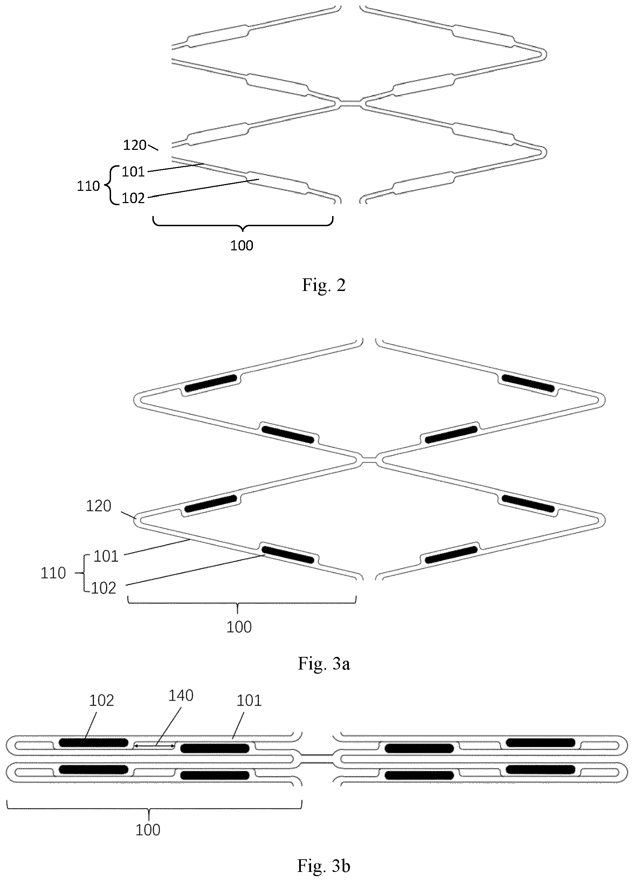

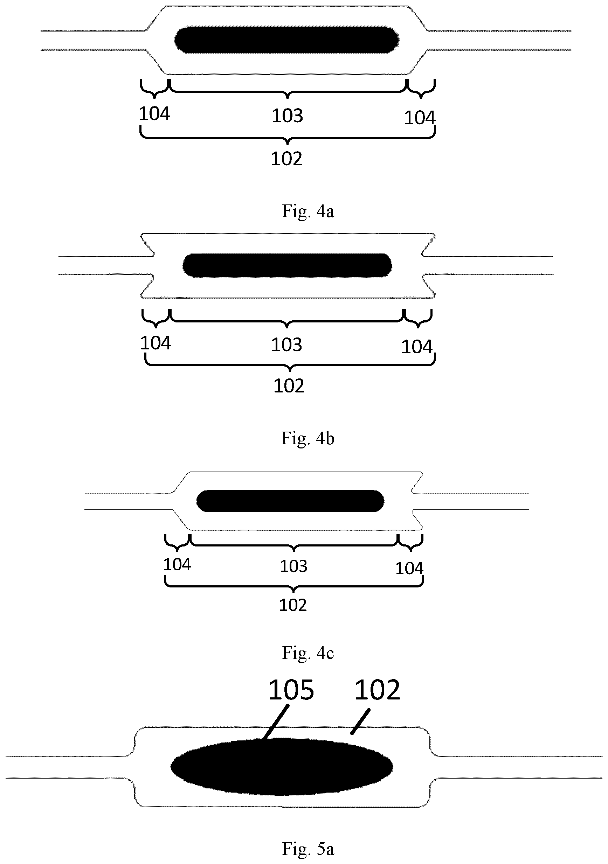

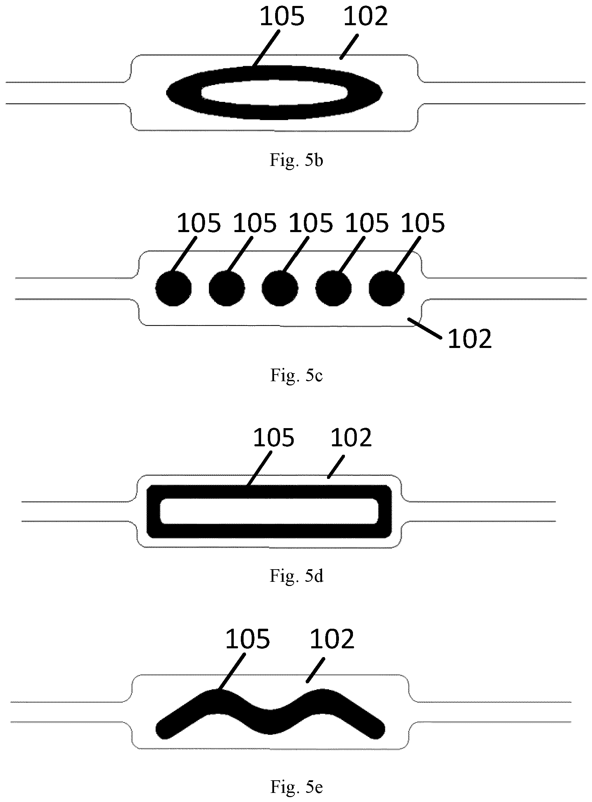

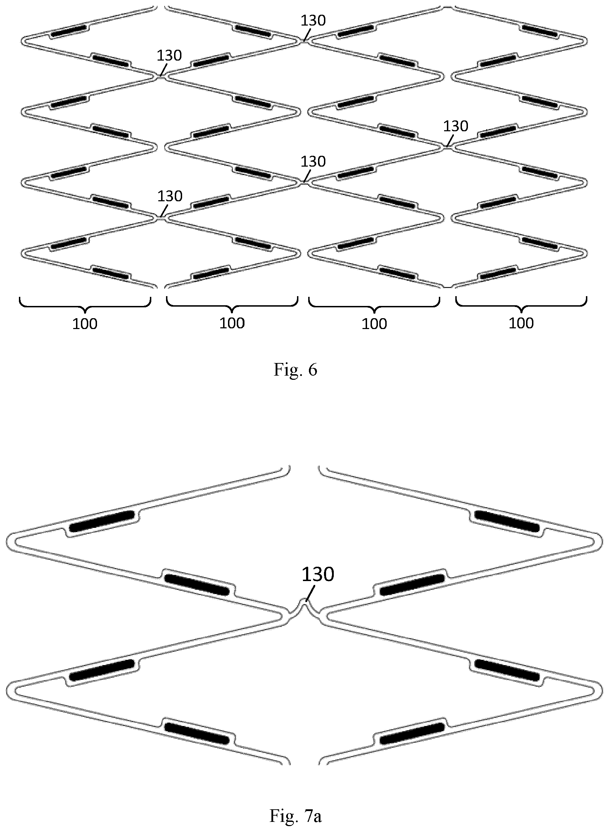

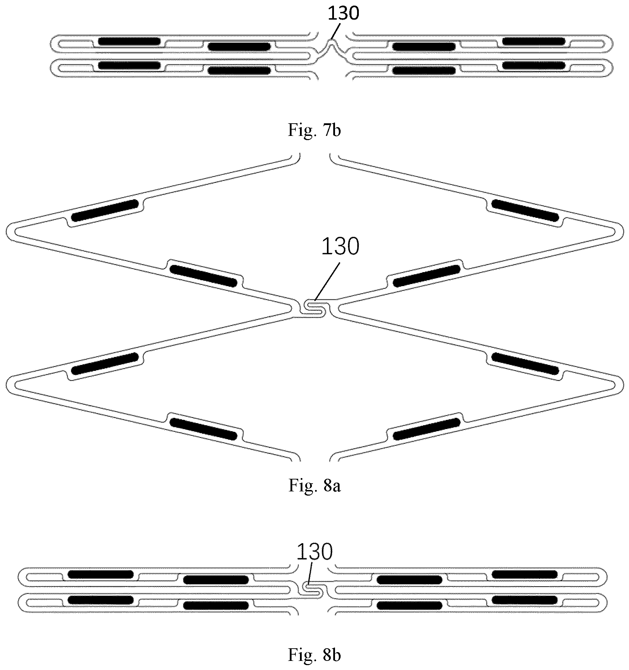

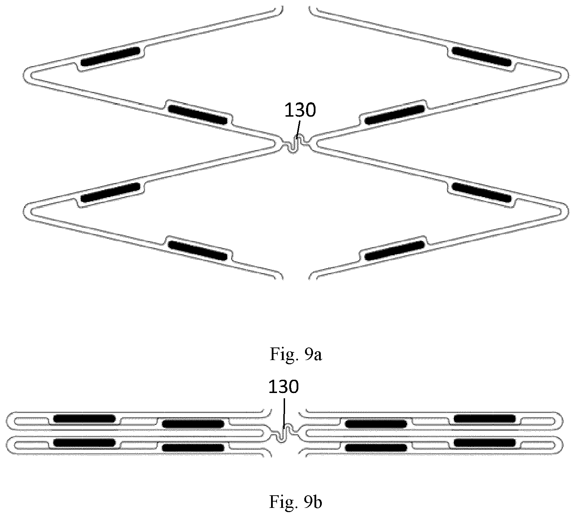

Reference will be made to Figs. 1a to 9b. Fig. 1a is a schematic partial view of a stent mesh according to an embodiment of the present invention. Fig. 1b is a schematic diagram showing the stent mesh of Fig. 1a in a collapsed configuration. Fig. 2 is a schematic partial view of a stent mesh according to another embodiment of the present invention. Fig. 3a is a schematic partial view of a stent mesh according to yet another embodiment of the present invention. Fig. 3b is a schematic diagram showing the stent mesh of Fig. 3a in a collapsed configuration. Fig. 4a is a schematic diagram showing a broadened section according to an embodiment of the present invention. Fig. 4b is a schematic diagram showing a broadened section according to another embodiment of the present invention. Fig. 4c is a schematic diagram showing a broadened section according to yet another embodiment of the present invention. Fig. 5a schematically illustrates a first optional implementation of a cavity pattern according to the present invention. Fig. 5b schematically illustrates a second optional implementation of a cavity pattern according to the present invention. Fig. 5c schematically illustrates a third optional implementation of a cavity pattern according to the present invention. Fig. 5d schematically illustrates a fourth optional implementation of a cavity pattern according to the present invention. Fig. 5e schematically illustrates a fifth optional implementation of a cavity pattern according to the present invention. Fig. 6 is a schematic partial view of a stent according to an embodiment of the present invention. Fig. 7a is a schematic partial view of a stent mesh according to the present invention, particularly showing an implementation of joints therein. Fig. 7b is a schematic diagram showing the stent mesh of Fig. 7a in a collapsed configuration. Fig. 8a is a schematic partial view of a stent mesh according to the present invention, particularly showing another implementation of joints therein. Fig. 8b is a schematic diagram showing the stent mesh of Fig. 8a in a collapsed configuration. Fig. 9a is a schematic partial view of a stent mesh according to the present invention, particularly showing yet another implementation of joints therein. Fig. 9b is a schematic diagram showing the stent mesh of Fig. 9a in a collapsed configuration.

As shown in Figs. 1a and 1b, in an embodiment, a stent is provided, which includes a plurality of stent meshes 100 each including plurality of stent struts 110, wherein the plurality of the stent struts 110 are sequentially connected end to end circumferentially around the stent mesh 100, and a joint 120 is formed at the connected ends of adjacent stent struts. The stent meshes 100 are configured to expand or collapse as a result of widening or narrowing of angles at the joints 120. Each stent strut 110 includes at least one main section 101 and at least one broadened section 102. The main section 101 has a width smaller than a width of the broadened section 102. The broadened sections 102 of adjacent stent struts 110 are staggered in an axial direction of the stent mesh 100. When the angles at the joints 120 are minimized, there are gaps 140 between the broadened sections 102 of adjacent stent struts 110 in the axial direction. In this embodiment, lengths are measured along axes of the stent struts. In this embodiment, the widths are measured in directions perpendicular to axes of the features being measured and both tangential to an outer circumferential surface of the stent mesh 100. In this embodiment, a lengthwise direction of the gaps 140 is defined to be parallel to a direction in which a length of the stent is measured and parallel to the axes of the stent struts. In Figs. 1a to 9b, the directions in which the widths are measured are perpendicular to both the axes of the features being measured and a line of sight of the viewer. The widthwise direction 200 and the lengthwise direction 300 are shown in Fig. 1a, and the gaps 140 are shown in Figs. 1b and 3b. Any other width, length or gap as mentioned herein below is defined in a similar fashion.

When the angles at the joints are in a range of 0° to 5°, metal coverage of the stent mesh ranges from 30% to 99 %. The metal coverage of the stent refers to a ratio of its total metal outer surface area to a total area of a vessel segment which is covered by the implanted stent. Specifically, it can be expressed as the formula: Metal Coverage = Ss/πDL × 100%, where Ss represents the metal outer surface area of the stent, D is an outer diameter of the covered segment, and L is a length of the covered segment. It would be appreciated that the metal coverage depends on the structure of the stent and thus can be increased by appropriately modifying the structure of the stent, optionally in an expanded or collapsed configuration.

It would be appreciated that, in the stent shown in Figs. 1a and 1b, there are a plurality of stent meshes 100. What is shown in Figs. 1a and 1b is only a part of th stent mesh 100 (as is the case of Figs. 3a to 3b and Figs. 6 to 9b described below), and the stent mesh 100 may be constructed from multiple replicas of the portion shown in Figs. 1a and 1b, which may be connected together therebetween. In various embodiments, each single stent mesh 100 may include 8 to 24, preferably, 12 to 18, stent struts 110. Likewise, the stent includes a plurality of (e.g., 2 to 18) stent meshes 100. Each stent mesh 100 is a ring-shaped structure made up of stent struts 110 which are connected end to end, and multiple such stent meshes 100 are connected axially to form the stent. When in an expanded configuration of the stent meshes 100 as shown in Fig. 1a, the stent can be used to support human tissue such as a blood vessel. When in a collapsed configuration of the stent meshes 100 as shown in Fig. 1b, the stent is suitable for passage through a limited space, such as a lumen of a blood vessel. The main sections 101 of the stent struts 110 allow the stent to overall occupy a smaller space, while the broadened sections 102 can ensure that metal coverage of the stent is large enough to enable desirable coverage of a plaque by the stent. The different widths of the broadened sections 102 and the main sections 101, as well as the staggered arrangement of the broadened sections 102, enable the stent to have desirable radial strength which can ensure that the stent is able to support a stenotic site while not causing damage to a blood vessel.

Referring to Fig. 2, in some embodiments, the stent provided herein may not have cavities. However, this is undesirable according to an analysis of drug-loaded stents performed by the inventors. Most existing drug loading techniques based on a self-expanding stent involve coating a surface of the stent with a drug layer or with drug-containing film. There is a known self-expanding stent, which is surface-coated with a polymer film formed by spraying drug-containing polymer nanofibers onto the stent surface. There is another known self-expanding luminal stent which is entirely or partially coated with a film attached thereto with a drug. There is yet another known stent consisting of a shape memory metal mesh with a coating film and a drug layer applied onto the film. All these techniques inevitably suffer from premature wear or peeling of the drug layer during crimping, loading and delivery of the self-expanding stent, and filling the drug into cavities formed in the stent is considered as a good solution to this problem. In fact, this design has found use in some stents for treating stenosis of extracranial arteries, such as coronary drug-eluting stents. Therefore, in some embodiments, cavities for containing a drug therein may be formed in the broadened sections, in order to increase drug loading capacity of the metal sections. The drug loading capacity can be achieved because: 1) the broadened sections 102 are wider and therefore satisfy the basic space requirements for drug loading; and 2) loading the drug in the cavity instead of coating it onto the stent surface circumvents the problem of premature wear or peeling of the drug during crimping, loading and delivery of the stent. This arrangement enables desirable space utilization and takes into account of both drug loading capacity and radial strength, allowing the stent to have a compact size in the collapsed configuration, which facilitates passage of the stent through a lesion site. Therefore, the stent can provide satisfactory ability to pass through a lesion site, desirable drug loading capacity and good radial strength.

With the above arrangement, the stent can have a radial dimension smaller than or equal to 0.0165 inches when in the collapsed configuration, which allows the stent to be used with the smallest available delivery microcatheters. In all embodiments, the stent may have an outer diameter of 0.3 to 0.7 mm when in the collapsed configuration, which allows the stent to be used with microcatheters with an inner diameter in the range of 0.013 inches to 0.027 inches.

As shown in Figs. 1a to 3b, when the angles at the joints 120 are minimized, there are gaps 140 between the broadened sections of adjacent stent struts 110 in the axial direction. The presence of the broadened sections 102 can increase the stent's metal coverage, while the presence of the axially spaced gaps 140 can prevent interference between the broadened sections 102 when the stent is being flexed, resulting in improved flexibility of the stent. In this way, the stent achieves a good tradeoff between metal coverage and flexibility, solving the problem of failing to provide both good metal coverage and desirable flexibility associated with conventional stents. Further, the present of the gaps 140 can improve compliance of the stent, enabling the stent to better comply with the geometry at a site in a blood vessel, without stretching the blood vessel or causing damage thereto.

In one embodiment, when the angle at the joint 120 is minimized, the gap 140 account for 0 to 90% of a length of the stent strut 110. In a preferred embodiment, when the angle at the joint 120 is minimized, the gap 140 account for 10% to 75% of the length of the stent strut 110. In a preferred embodiment, when the angle at the joint 120 is minimized, the gap 140 account for 20% to 50% of the length of the stent strut 110. In some specific embodiments, when the angle at the joint 120 is minimized, the gap 140 account for 1%, 2%, 5%, 10%, 15%, 20%, 25%, 30%, 35%, 40%, 45%, 50%, 55%, 60%, 65%, 70%, 75%, 80%, 85%, 90% or another percentage of the length of the stent strut 110. In order to enable the stent to have both good metal coverage and desirable flexibility, in an even preferred embodiment, when the angle at the joint 120 is minimized, the gap 140 account for 2.5% to 12.5% of the length of the stent strut 110.

In various embodiments, the stent struts 110 may have a length ranging from 1 mm to 2.5 mm. In a preferred embodiment, the length of the stent struts 110 may range from 1.2 mm to 1.5 mm. In various embodiments, the gaps 140 may have a length ranging from 0.1 mm to 0.3 mm. In a preferred embodiment, the length of the gaps 140 may range from 0.15 mm to 0.25 mm. In some specific embodiments, the length of the gaps 140 may be 0.1 mm, 0.12 mm, 0.15 mm, 0.18 mm, 0.2 mm, 0.22 mm, 0.25 mm, 0.27 mm, 0.3mm or the like.

In various embodiments, the stent struts 110 may have a width ranging from 20 mm to 150 mm. In various embodiments, the main sections 101 may have a width ranging from 30 µm to 50 µm. In various embodiments, the broadened sections 102 may have a width ranging from 90 µm to 120 µm.

The inventors carried out tests with various ratios of total length of the broadened section(s) 102 to length of the stent strut 110. All the tested stents had an outer diameter of 0.53 mm in the collapsed configuration, a total length of 9.68 mm in the collapsed configuration, a stent strut thickness of 0.07 mm and a stent mesh count of 6. Starting with all these same initial dimensions in the collapsed configuration, the stents expanded to a radial dimension of 3 mm, when their radial strength and metal coverage were measured. The results are shown in Table 1.

**Table 1 Dependence of Radial Strength and Metal Coverage on Ratio of Broadened Sections**

| No. | ratio of total length of broadened section(s) to length of stent strut (%) | ratio of total length of broadened section(s) in adjacent stent struts to length of stent strut (%) | radial strength (kPa) | metal coverage (%) |
|---|---|---|---|---|
| 1 | 0 | 0 | 6.77 | 7.51 |
| 2 | 0 | 100 | 10.5 | 13.12 |
| 3 | 50 | 50 | 9 | 11.31 |
| 4 | 10 | 90 | 9.1 | 12.85 |
| 5 | 5 | 5 | 6.92 | 8.62 |
| 6 | 5 | 95 | 9.7 | 12.93 |
| 7 | 30 | 30 | 8.71 | 11.11 |
| 8 | 15 | 15 | 7.23 | 8.93 |
| 9 | 20 | 20 | 7.56 | 9.24 |
| 10 | 25 | 75 | 9.23 | 12.84 |

As can be seen from the data in Table 1, in comparison to the results of Test No. 1 serving as a control test, the stents with ratios of total length of the broadened section(s) 102 in one stent strut to length of the stent strut 110 ranging from 5% to 95% exhibit both desirable radial strength and sufficiently large metal coverage.

As can be also seen from the data in Table 1, the ratio of total length of the broadened section(s) 102 in one stent strut to length of the stent strut 110 is preferably 15% to 75%, more preferably 20% to 50%. In some specific embodiments, the ratio of total length of the broadened section(s) 102 in one stent strut to length of the stent strut 110 may be 5%, 10%, 15%, 20%, 25%, 30%, 35%, 40%, 45%, 50%, 55%, 60%, 65%, 70%, 75%, 80%, 85%, 90%, 95% or the like.

Additionally, the ratio of the total length of the broadened section(s) 102 in adjacent stent strut 110 to the length of the stent strut 110 does not exceed 100%. As also can be seen from the data in Table 1, a ratio of the total length of the broadened section(s) 102 in adjacent stent strut 110 to the length of stent strut 110 may be the same or different.

As also can be seen from the data in Table 1, the stents show radial strength of 1 kPa to 100 kPa.

In various embodiments, the length of the stent struts 110 may range from 1 mm to 2.5 mm. In a preferred embodiment, the length of the stent struts 110 may range from 1.2 mm to 1.5 mm. The stent struts 110 may be generally linear in shape, or may have one or more curved portions, but they extend overall in the lengthwise direction.

In various embodiments, the width of thee stent struts 110 may range from 20 mm to 150 mm. In various embodiments, the width of the main sections 101 may range from 30 µm to 50 µm. In various embodiments, the width of the broadened sections 102 may range from 90 µm to 120 µm.

The metal coverage of the stent is determined by its structure and the angles at the joints 120. When the structure of the stent is uniform, the angle refers to an angle between adjacent stent struts 110 connected to joint 120. When the angles at the joints 120 are 0°, the angle between adjacent stent struts 110 connected to joint 120 is 0°, the stent is the aforementioned collapsed configuration, resulting in large metal coverage of the stent. When the angles at the joints 120 are 30° to 120°, the stent is the aforementioned expanded configuration where it shows reduced metal coverage. In the collapsed configuration, at a given total metal surface area of the stent and a given thickness of the struts therein, larger metal coverage means a smaller outer diameter of the stent, which allows the stent to be more easily advanced in a blood vessel and to be delivered to a more distant target site or a target site in a narrower blood vessel. In the expanded configuration, larger metal coverage allows the stent to more desirably cover a plaque at a lesion site, providing better therapeutic efficacy. The design with alternating wide and narrow strut sections enables the stent to have effectively improved metal coverage while exhibiting satisfactory radial strength. Metal coverage of different stent meshes 100 at various angles is shown in Table 2.

**Table 2 Metal Coverage of Different Stent Meshes at Various Angles**

| No. | Metal Coverage of Single Stent Mesh (%) | | | | | |
|---|---|---|---|---|---|---|
| | Angle at 0° | Angle at 5° | Angle at 30° | Angle at 60° | Angle at 90° | Angle at 120° |
| Sample 1 | 38.44 | 33.86 | 8.81 | 4.78 | 4.16 | 4.03 |
| Sample 2 | 53.50 | 47.12 | 11.18 | 6.65 | 5.79 | 5.61 |
| Sample 3 | 94.39 | 83.14 | 11.88 | 11.74 | 10.21 | 9.89 |
| Sample 4 | 96.45 | 84.95 | 12.02 | 11.99 | 10.43 | 10.11 |
| Sample 5 | 96.14 | 84.67 | 12.06 | 11.96 | 10.40 | 10.08 |
| Sample 6 | 95.82 | 84.39 | 12.89 | 11.92 | 10.37 | 10.04 |

As can be seen from the data in Table 2, when the stent is in a configuration where the angles at the joints in the stent range from 0° to 5°, each single stent mesh 100 has metal coverage of 30% to 99%. As can be also seen from Table 2, in said configuration of the stent where the angles at the joints in the stent range from 0° to 5°, the single stent mesh 100 is preferred to have metal coverage of 80% to 99%.

As can be seen from the data in Table 2, the angles at the joints 120 may range from 0° to 120°. In some other embodiments, the angles at the joints 120 may range from 0° to 140°. When the angles at the joints 120 are 0° or almost 0°, the stent is in the collapsed configuration. This can be achieved by crimping the stent into a guide sheath before loading into a microcatheter. The stent remains in the collapsed configuration after it is loaded into the microcatheter and during the subsequent delivery therein. Notably, it is not the case that the stent is in the collapsed configuration only when the angles at the joints 120 are 0°. Depending on an inner diameter of the microcatheter, the stent may be loaded therein with various angles at the joints 120. A larger inner diameter of the microcatheter will lead to larger angles at the joints 120. For example, the angles at the joints 120 may be 0° to 5° in the collapsed configuration of the stent. When the angles at the joints 120 lie between 30° and 120°, the stent comprises the expanded configuration. In some embodiments, when the stent expands to an outer diameter of 2 mm to 3 mm, the angles at the joints 120 are in the range of 30° to 90°. It is noted that the expanded configuration is not necessarily a native configuration of the stent where it fully expands. Rather, it may only expand to a predetermined outer diameter in the expanded configuration. For example, when placed in a tube with an inner diameter of 2 mm to 3 mm, the stent may expand to an outer diameter of 2 mm to 3 mm in the expanded configuration. At this time, the angles at the joints 120 are greater than those in the collapsed configuration. After being taken out of the tube or transferred into a tube with a greater inner diameter, the stent may further expand, leading to an increase in the angles at the joints 120.

When the angles at the joints are in the range of 5° to 30°, the single stent mesh shows metal coverage of 5% to 90%.

As can be seen from the data in Table 2, when the angles at the joints are in the range of 30° to 90°,the single stent mesh shows metal coverage of 4% to 15%. As can be also seen from the data in Table 2, the metal coverage of each single stent mesh 100 is preferred to be 8% to 15% when the angles at the joints in the stent is in the range of 30° to 90°.

When the angles at the joints are in the range of 90° to 140°, the single stent mesh shows metal coverage of 3% to 12%.

Apart from the metal coverage of the individual stent meshes 100, overall metal coverage of the stent may also depend on linking struts 130 between the stent meshes. The longer the linking struts 130, the greater the distant between the stent meshes 100. Due to the presence of the linking struts 130, the overall metal coverage of the stent is smaller than the metal coverage of the individual stent meshes 100. When the linking struts 130 are equally long and of the same structure, the greater the number of linking struts between the stent meshes 100, the greater the metal coverage of the stent. In addition, the metal coverage of the stent may also depend on the structure of the linking struts. In the embodiments provided herein, when the angles at the joints are in the range of 0° to 5°, the overall metal coverage of the stent is 20% to 60%, preferably 35 to 60%; when the angles at the joints are in the range of 5° to 30°, the overall metal coverage of the stent is 5% to 45%, preferably 8% to 40%; when the angles at the joints are in the range of 30° to 90°, the overall metal coverage of the stent is 3% to 15%, preferably 8% to 15%; and when the angles at the joints are in the range of 90° to 140°, the overall metal coverage of the stent is 2% to 15%, preferably 5% to 15%.

In various embodiments, each stent strut 110 may include 1 to 10 broadened sections 102. Preferably, each stent strut 110 includes one broadened section 102. This enables good space utilization and ensures large metal coverage and/or a sufficient drug load. Moreover, each stent strut 110 may include 1 to 11 main sections 101 alternating with the broadened section(s) 102 in the same stent strut 110. Preferably, each stent strut 110 includes 2 main sections 101. A ratio of width of the main section 101 to width of the broadened section 102 may range from 1/4 to 2/3. Preferably, the ratio is 1/3, in order for good space utilization to be achieved. Adjacent stent struts 110 may include the same or different numbers of broadened sections 102. Each stent strut 110 may include one or more broadened sections 102. Referring to Fig. 1a, in one embodiment, one of the stent struts 110 includes three main sections 101 and two broadened sections 102 alternating with the broadened sections 102. Each broadened section is located between two main sections. Moreover, in the same stent mesh 100, an adjacent stent strut includes two main sections 101 and one broadened section 102 located between the main sections. With continued reference to Fig. 2, in a preferred embodiment, each stent strut 110 in a stent mesh 100 includes two main sections 101 and one broadened section 102 located between the main sections.

Each stent mesh 100 further includes bends 121 for connecting the stent struts 110. In various embodiments, a ratio of width of the main section 101 to width of the bend 121 may range from 1/2 to 3/4. Preferably, the ratio is 3/5, which can prevent breakage at the joints 120 while enabling the stent to have low strength.

In various embodiments, for each stent strut 110, a ratio of length of the stent strut 110 to length of the shortest main section 101 therein may be greater than or equal to 10/3, preferably 6/1.

In various embodiments, a ratio of length of the stent strut 110 to length of the bend 121 may be greater than or equal to 8. Preferably, this ratio is 12, which can prevent breakage at the joints 120 while enabling the stent to have low strength.

Preferably, when the angles at the joints 120 are minimized, in each stent strut 110, each broadened section 102 does not overlap with main section 101 in an adjacent stent strut 110 connected to the same joint 120. During design, backward inference may be used to calculate a dimension of the broadened sections 102 from a radial distance between the main sections 101 in adjacent stent struts 110 at the minimized angles, under the premise of ensuring overall structural strength and a designed service lifespan. This can additionally enhance space utilization and enable the stent to have an even smaller overall size. In some embodiments, the main sections 101 in different pairs of adjacent stent struts 110 connected to respective joints 120 may be spaced by different radial distances when the angles are minimized. In this case, the widths of the broadened sections 102 in the stent struts 110 may be individually designed in order to achieve good space utilization. Alternatively, all the broadened sections 102 may be designed with the same width, according to the smallest one of the radial distances of all the pairs of adjacent stent struts 110 at the minimized angles. This represents a low-cost design approach.

It would be appreciated that the above-discussed embodiments are preferred, but in some special cases, when the angles at the joints 120 are minimized, in each pair of adjacent stent struts 110 connected to the same joint 120, the radial distance between their main sections 101 may also be smaller than the widths of protrusion of the broadened sections 102 relative to the main sections 101on one side. This can also achieve good space utilization.

As shown in Fig. 2, in one embodiment, the broadened sections 102 have margins of the same width beyond the main sections 101 at opposite sides of the stent struts along the lengthwise direction thereof. Center axes of the broadened sections 102 coincide with Center axes of the stent struts 110, optionally with offsets within a manufacturing tolerance range.

In some other embodiments, the broadened sections 102 may have margins of different widths beyond the main sections 101 at opposite sides of the stent struts along the lengthwise direction thereof. For example, as shown in Figs. 3a and 3b, in one embodiment, the broadened sections 102 are flush with the main sections 101 at one side of the stent strut along the lengthwise direction thereof. Preferably, in the expanded configuration of the stent, each pair of adjacent stent struts 110 connected to the same joint 120 forms a V-shaped structure, wherein the sides of the adjacent stent struts 110, at which the broadened sections 102 are flush with the main sections 101, are both located at inner side or outer side of the V-shaped structure.

In other embodiments, the sides of the adjacent stent struts 110, at which the broadened sections 102 are flush with the main sections 101 may be located at sides of the V-shaped structure facing the same direction. In other words, in the expanded configuration of the stent, adjacent stent struts 110 connected to the same joint 120 forms a V-shaped structure, wherein the sides of the adjacent stent struts 110, at which the broadened sections 102 are flush with the main sections 101, are not both located at inner or outer sides of the V-shaped structure. That is, one side of the adjacent stent struts 110, at which the broadened sections 102 are flush with the main sections 101, is located at the inner edges of the V-shaped structure, while the other side of the adjacent stent struts 110, at which the broadened sections 102 are flush with the main sections 101, is located at the outer side of the V-shaped structure.

Referring to Figs. 4a to 4c, each broadened section 102 includes a main segment 103 and transition segments 104 each joining the main segment 103 to one main section 101. The transition segments 104 may comprise any of various shapes. For example, in one embodiment, the transition segments 104 are each tapered from the main segment 103 toward the main section 101 (see Fig. 4a). In an alternative embodiment, the transition segments 104 each include two outwardly open transition notches on opposite sides of the main section 101. The transition segments 104 are as wide as the main segment 103, and the openings of the transition notches are tapered from the main section 101 toward the main segment 103 (see Fig. 4b). In another alternative embodiment, out of the transition segments 104 of all the broadened sections 102, at least some have at least one of the above-discussed shapes (i.e., any of the examples shown in Figs. 4a, 4b and 4c). It would be appreciated that the transition segments 104 may also be configured otherwise. In some other embodiments, for example, in the embodiment shown in Fig. 3a, each broadened section 102 may only include the main segment 103 and, in this case, the main segment 103 is directly joined to the main sections 101. It is noted that, as shown in Figs. 4a to 4c, in case of the broadened sections 102 including the transition segments 104, the gaps at different locations may have different lengths. As long as there is any gap 140 along an arbitrary axial location of the stent struts 110, a space is provided to accommodate deformation of the stent during narrowing of the angles at the joints 120, resulting in an increase in the stent's flexibility. In a preferred embodiment, when the angles at the joints 120 are minimized, along any axial location of the stent, length of a gap accounts for 0 to 90% of the length of strut.

Since the broadened sections have a relatively large surface area, cavities can be formed therein, in which a drug or radiopaque agent may be filled to increase the stent's drug loading capacity or radiopacity. Referring to Figs. 5a to 5e, the broadened sections 102 may include cavities 105, the cavity may be formed by cavity pattern(s), the cavity pattern comprises at least one of a solid pattern, a hollow pattern, a continuous pattern and a discontinuous pattern. The cavity may have a shape comprising at least one of a cylinder, a cuboid and a triangular prism. The cavity may have a transverse cross-sectional shape comprising at least one of a circular shape, an elongate shape, a polygonal shape, a corrugated shape, an annular shape and an irregular shape. The cavity may have a longitudinal cross-sectional shape comprising at least one of an arcuate shape, a quadrilateral shape and a triangular shape. Notably, the transverse cross-sectional shape refers to the shape of a cross-section taken parallel to an outer surface of the broadened section 102 in which the cavity is formed, and the longitudinal cross-sectional shape refers to the shape of a cross-section taken perpendicular to both the outer surface and a lengthwise direction of the broadened section 102. The cavities 105 are configured for a drug or radiopaque agent to be filled therein. In some embodiments, the cavities 105 may be through slots extending through the stent strut 110 across its entire thickness. In some other embodiments, the cavities 105 may be blind holes extending through a partial thickness of the stent struts 110. It would be appreciated that Figs. 4a to 4c are meant to particularly exemplify and illustrate the transition segments 104, and Figs. 5a to 5e are meant to particularly exemplify and illustrate the cavities 105. It is in no way intended that when the transition segments 104 are configured as shown in any of Figs. 4a to 4c, the cavities 105 have to be shaped as shown in the same figure, or that when the cavities are configured as shown in any of Figs. 5a to 5e, the transition segments 104 have to be shaped as shown in the same figure. These implementations may be combined in any combination.

In various embodiments, a ratio of a maximum width of the cavity pattern to the width of the broadened section 102 may be in the range of 1/3 to 4/5. Preferably, this ratio is 1/2, because it can enable good space utilization and ensure a sufficient drug load. The width of the cavity pattern is measured in the same direction as the width of the broadened sections 102 is measured.

Referring to Fig. 1a, in one embodiment, the stent includes at least two stent meshes 100 that are axially connected. The stent may include 4 to 18 stent meshes 100, preferably 6 to 10 stent meshes 100. When the stent expands to an outer diameter of 2 mm to 3 mm, each stent mesh may have a length of 1 mm to 1.8 mm. The stent meshes may be connected to each other in any suitable way. In a preferred embodiment, referring to Fig. 6, the stent includes linking struts 130, and the joints 120 in adjacent stent meshes 100 are aligned and interconnected by the linking struts 130. In the embodiment shown in Fig. 6, the joints 120 in adjacent stent meshes 100 are aligned, and the two stent meshes 100 are in mirrored symmetry with each other. Additionally, referring to Fig. 6, along circumference of the stent, a non-connected pair of joints 120 is interposed between each adjacent connected pair of joints 120. This arrangement can facilitate the widening or narrowing of the angles at the joints and enhance space utilization. It would be appreciated that, in alternative embodiments, the joints 120 may be not aligned and the linking struts may be not located at the joints 120. Moreover, when the joints 120 are aligned and connected by the linking struts 130, the connections may be made in another way than as discussed above. For example, all pairs of aligned joints 120 may be each connected with a linking strut 130. Alternatively, the pairs of aligned joints 120 may be connected with the linking struts 130 at an interval of two non-connected pairs circumferentially around the stent. Still other arrangements are possible.

In various embodiments, a ratio of width of the main section 101 to width of the linking strut 130 may be in the range of 1/2 to 1. This ratio is preferred to be 1/2, in order to satisfactorily prevent breakage of the linking struts 130.

Referring to Figs. 1a to 1b and 7a to 9b, the linking struts 130 may have a shape comprising at least one of a linear shape, a corrugated shape, a serrated shape, a circular shape, an annular shape, a "Ω"-like shape and an "S"-like shape. The linking struts 130 are preferred to be non-linear in shape, for example, corrugated shape, "Ω"-shaped or "S"-shaped, because these shapes can facilitate overall collapse and expansion of the stent. It would be appreciated that various implementations of the linking struts 130 may be combined with those of the above-discussed cavities 105, transition segments 104 and broadened sections 102 in any combination.

In other embodiments, cavities may also be formed in the linking struts 130, though this is not favorable from the point of view of overall structural reliability.

In some embodiments, the stent may be formed of a cobalt-based alloy, a magnesium alloy, a nickel-titanium alloy, stainless steel or the like, or a combination thereof.

There is also provided herein a drug-loaded stent differing from the stent as defined above mainly in including cavities 105, in which a drug or radiopaque agent is filled.

For details of other features of the drug-loaded stent, reference can be made to the above description of this specification in connection with the stent.

This invention provides a stent including at least one stent mesh 100 each including a plurality of stent struts 110, wherein the plurality of the stent struts 110 are sequentially connected end to end, and a joint 120 is formed at the connected ends of adjacent stent struts 110. The stent mesh 100 is configured to expand or collapse as a result of widening or narrowing of angles at the joints 120. Each stent strut 110 includes at least one main section 101 and at least one broadened section 102 alternating with the at least one main section 101. The main section 101 has a width smaller than that of the broadened section 102. In each stent mesh 100, the broadened sections 102 of adjacent stent struts 110 are staggered along an axis of the stent mesh 100. This invention also provides a drug-loaded stent of a similar structure. The different widths of the broadened sections 102 and the main sections 101, as well as the staggered arrangement of the broadened sections 102, enable sensible space utilization and achieve a good tradeoff between metal coverage and radial strength. In this way, the stent is allowed to have a compact size when in a collapsed configuration, which facilitates passage of the stent through a lesion site. Additionally, there are gaps between the broadened sections 102 of adjacent stent struts 110, which impart increased flexibility to the stent. As a result, the stent is readily flexible within a blood vessel and can be more easily delivered to a target site. Moreover, at the target site, the stent can better comply with the geometry of the blood vessel, without stretching the wall of the blood vessel or causing damage thereto. Thus, the problem of failing to provide both good metal coverage and desirable flexibility associated with conventional stents is overcome.

The description presented above is merely that of some preferred embodiments of the present invention and is not intended to limit the scope thereof. Any and all changes and modifications made by those of ordinary skill in the art based on the above teachings fall within the scope as defined in the appended claims.

## Claims

1. A stent, comprising at least one stent mesh, wherein each stent mesh comprises a plurality of stent struts sequentially connected circumferentially around the stent mesh, wherein the plurality of the stent struts are sequentially connected end to end, and a joint is formed at the connected ends of adjacent stent struts, and wherein the stent mesh is configured to expand or collapse as a result of widening or narrowing of an angle at the joint,
wherein each stent strut comprises at least one main section and at least one broadened section that are alternately arranged with the at least one main section, wherein the main section comprises a width smaller than a width of the broadened section, wherein: in each stent mesh, the broadened sections of the adjacent stent struts are staggered along an axis of the stent mesh; and when the angle at each joint is minimized, at least one gap is provided between the broadened sections of the adjacent stent struts along the axis.

2. The stent according to claim 1, wherein when the angle at the joint is minimized, a length of the gap accounts for 0 to 90% of a length of the stent strut.

3. The stent according to claim 2, wherein when the angle at the joint is minimized, the length of the gap accounts for 10% to 75% of the length of the stent strut.

4. The stent according to claim 3, wherein when the angle at the joint is minimized, the length of the gap accounts for 20% to 50% of the length of the stent strut.

5. The stent according to claim 1, wherein when the angle at the joint is minimized, the gap has a length of 0.1 mm to 0.3 mm.

6. The stent according to claim 1, wherein the angle at each joint ranges from 0° to 140°.

7. The stent according to claim 1, wherein when the angle at each joint is in a range of 0° to 5°, a metal coverage of the stent mesh ranges from 30% to 99%.

8. The stent according to claim 1, wherein when the angle at each joint is in a range of 5° to 30°, a metal coverage of the stent mesh ranges from 5% to 90%.

9. The stent according to claim 1, wherein when the angle at each joint is in a range of 30° to 90°, a metal coverage of the stent mesh ranges from 4% to 15%.

10. The stent according to claim 9, wherein when the angle at each joint is in the range of 30° to 90°, the metal coverage of the stent mesh ranges from 8% to 15%.

11. The stent according to claim 1, wherein when the angle at each joint is in a range of 90° to 140°, a metal coverage of the stent mesh ranges from 3% to 12%.

12. The stent according to claim 1, wherein when the angle at each joint is in a range of 0° to 5°, a metal coverage of the stent ranges from 20% to 60%.

13. The stent according to claim 1, wherein when the angle at each joint is in a range of 5° to 30°, a metal coverage of the stent ranges from 5% to 45%.

14. The stent according to claim 1, wherein when the angle at each joint is in a range of 30° to 90°, a metal coverage of the stent ranges from 3% to 15%.

15. The stent according to claim 1, wherein when the angle at each joint is in a range of 90° to 140°, a metal coverage of the stent ranges from 2% to 15%.

16. The stent according to claim 1, wherein the stent comprises a radial strength of 1 kPa to 300 kPa.

17. The stent according to claim 1, wherein the stent strut comprises two main sections and one broadened section located between the two main sections.

18. The stent according to claim 1, wherein the stent mesh comprises 8 to 24 stent struts.

19. The stent according to claim 1, wherein at least one of the broadened sections comprises a cavity.

20. The stent according to claim 19, wherein the broadened section comprises 1 to 10 cavities.

21. The stent according to claim 19, wherein a longitudinal cross-sectional shape of the cavity comprises at least one of an arcuate shape, a quadrilateral shape and a triangular shape.

22. The stent according to claim 19, wherein a transverse cross-sectional shape of the cavity comprises at least one of a circular shape, an elongate shape, a polygonal shape, a corrugated shape, an annular shape and an irregular shape.

23. The stent according to claim 19, wherein the cavity is configured for a drug or radiopaque agent to be filled therein.

24. The stent according to claim 1, wherein when the angle at the joint is minimized, in adjacent stent struts connected to a same joint, the broadened section of one of the stent struts does not overlap with the main section of the other one of the stent struts.

25. The stent according to claim 1, wherein in at least one of stent strut, the broadened section has margins of a same width or different widths beyond the main section at opposite sides of the stent strut along a lengthwise direction thereof.

26. The stent according to claim 1, wherein in at least one of stent strut, the broadened section is flush with the main section at one side of the stent strut along a lengthwise direction thereof.

27. The stent according to claim 26, wherein in an expanded configuration of the stent, the adjacent stent struts connected to a same joint form a V-shaped structure, wherein the sides of the adjacent stent struts, at which the main sections are flush with the broadened sections, are both located at an inner side or an outer side of the V-shaped structure.

28. The stent according to any one of claims 1 to 27, wherein the stent comprises at least two stent meshes that are axially connected.

29. The stent according to claim 28, wherein the stent comprises at least one linking strut, and wherein the joints in adjacent stent meshes are connected through the at least one linking strut.

30. The stent according to claim 29, wherein a shape of the linking strut comprises at least one of a linear shape, a corrugated shape, a serrated shape, a circular shape, an annular shape, a "Ω"-like shape and an "S"-like shape.

31. A drug-loaded stent, comprising at least one stent mesh wherein each stent mesh comprises a plurality of stent struts sequentially connected circumferentially around the stent mesh, wherein the plurality of the stent struts are sequentially connected end to end, and a joint is formed at the connected ends of adjacent stent struts, and wherein the stent mesh is configured to expand or collapse as a result of widening or narrowing of an angle at the joint,
wherein each stent strut comprises at least one main section and at least one broadened section that are alternately arranged with the at least one main section, wherein the main section has a width smaller than a width of the broadened section, wherein: in each stent mesh, the broadened sections of the adjacent stent struts are staggered along an axis of the stent mesh; at least one broadened section comprises a cavity configured for a drug to be filled therein; and when the angle at each joint is minimized, at least one gap is provided between the broadened sections of the adjacent stent struts along the axis.
